(19) Europäisches Patentamt
European Patent Office
Office européen des brevets

(11) Veröffentlichungsnummer: **0 020 964**
**A1**

(12) # EUROPÄISCHE PATENTANMELDUNG

(21) Anmeldenummer: **80102515.6**

(22) Anmeldetag: **08.05.80**

(51) Int. Cl.³: **C 07 D 231/12**, C 07 D 231/14, C 07 D 231/16, C 07 D 231/18

(30) Priorität: **02.06.79 DE 2922591**

(43) Veröffentlichungstag der Anmeldung: **07.01.81 Patentblatt 81/1**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Aders, Wolf-Karlo, Dr., Ernst-Merk-Strasse 13, D-6701 Ellerstadt (DE)**
Erfinder: **Mangold, Dietrich, Dr., Hermann-Walker-Strasse 49, D-6903 Neckargemuend (DE)**
Erfinder: **Wahl, Josef, Bitzstrasse 25, D-6707 Schifferstadt (DE)**
Erfinder: **Rotermund, Gerhard W., Dr., Landfriedstrasse 3, D-6900 Heidelberg (DE)**

(54) **Verfahren zur Herstellung von Pyrazolen.**

(57) Herstellung von Pyrazolen durch Umsetzung von 1,3-Dicarbonylverbindungen mit Hydrazinen und unterschüssiger Säure bei einem pH-Wert zwischen 0 und 6,9. Die nach dem Verfahren der Erfindung herstellbaren Pyrazole sind wertvolle Ausgangsstoffe für die Herstellung von Farbstoffen, Pflanzenschutzmitteln und Pharmazeutika.

EP 0 020 964 A1

## Verfahren zur Herstellung von Pyrazolen

Die Erfindung betrifft ein neues Verfahren zur Herstellung von Pyrazolen durch Umsetzung von 1,3-Dicarbonylverbindungen mit Hydrazinen und unterschüssiger Säure bei einem pH-Wert zwischen 0 und 6,9.

Es sind aus Rodd, Chemistry of Carbon Compounds, Band IVa (Elsevier, N.Y., 1957), Seiten 246 bis 249, zahlreiche Synthesen der Pyrazole bekannt, z.B. durch Umsetzung von Hydrazinen mit 1,3-Dicarbonylverbindungen, Dehydratation von Hydrazonen der ß-Ketoester, Cyclisierung von Hydrazonen der α-Cyanoketone, Umsetzung von Aldehydphenylhydrazonen mit ß-Ketoestern in Gegenwart von Zinkchlorid, Kondensation von α-Halogenhydrazonen mit Natrium-Ketoverbindungen oder von Diazoverbindungen mit Acetylenderivaten. Die bedeutenderen Synthesewege führen über die Umsetzung von Derivaten von 1,3-Dicarbonylverbindungen mit Hydrazin oder Hydrazinderivaten in saurer, wäßriger Lösung oder mit Salzen des Hydrazins oder von Hydrazinderivaten zu verdünnten wäßrigen Lösungen von Pyrazolsalzen. So zeigt J.A.C.S., Band 71 (1949), Seite 3 997, die stöchiometrische Umsetzung von 1,1,3,3-Tetraäthoxypropan mit Hydrazindihydrochlorid in einem Wasser/Äthanol-Gemisch. Die Lösung wird dann eingedampft, der Rückstand mit Wasser aufgenommen und aus der Lösung durch Natriumbicarbonat das Pyrazol aus seinem Salz freigesetzt. Das Gemisch wird filtriert, das Filtergut gründlich mit Äther gewaschen und das Ätherfiltrat zur Extraktion des wäßrigen Filtrats verwendet.

Da ja bei diesen Synthesen von Salzen des Hydrazins (oder seiner Derivate) in Lösung ausgegangen wird, ist es unvermeidlich, nach Verwendung z.B. anorganischer Basen, stöchiometrische Mengen an Abfallsalzen zu erzeugen. Bei der Entsorgung im großtechnischen Maßstab führen diese Salze zu

WB/BL

erheblichen Abwasser- und Umweltschutzproblemen. Nach der vorgenannten Arbeitsmethode (J.A.C.S., loc. cit.) werden beispielsweise pro kg hergestellten Pyrazols mehr als 2 kg Kochsalz frei.

Es wurde nun gefunden, daß man Pyrazole der Formel

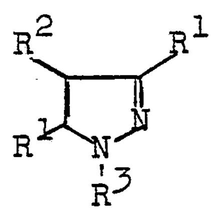 I,

worin die einzelnen Reste $R^1$, $R^2$ und $R^3$ gleich oder verschieden sein können und jeweils ein Wasserstoffatom, einen aliphatischen, araliphatischen oder aromatischen Rest bedeuten, $R^2$ auch ein Halogenatom, den Rest -CN oder den Rest $-O-R^4$ bezeichnet, $R^3$ auch für den Rest $\overset{O}{\overset{\parallel}{-C}}-R^4$ steht, $R^4$ einen aliphatischen, araliphatischen oder aromatischen Rest bedeutet, durch Umsetzung von 1,3-Dicarbonylverbindungen mit Hydrazinen in Gegenwart von Wasser und Säure vorteilhaft erhält, wenn man 1,3-Dicarbonylverbindungen der Formel

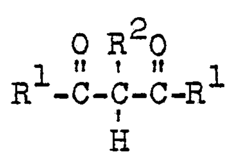 II,

worin $R^1$ und $R^2$ die vorgenannte Bedeutung besitzen, mit Hydrazinen der Formel

$$R^3-HN-NH_2 \quad \text{III},$$

worin $R^3$ die vorgenannte Bedeutung besitzt, in Gegenwart von weniger als 2 Äquivalenten Säure je Mol Hydrazin III bei einer Temperatur von -10 bis +100°C umsetzt, wobei der

pH-Wert des Reaktionsgemisches während der Reaktion zwischen 0 und 6,9 beträgt.

Weiterhin wurde gefunden, daß man das Verfahren nach der Erfindung vorteilhaft ausführt, wenn man in dem Reaktionsgemisch den Anteil des Pyrazols I in Gestalt des Pyrazolsalzes mit zusätzlichem Hydrazin III zu freiem Pyrazol I umsetzt.

Im Hinblick auf den Stand der Technik liefert das Verfahren nach der Erfindung überraschend auf einfacherem und wirtschaftlicherem Wege Pyrazole in besserer Ausbeute und Reinheit. Wesentliche Säuremengen und dementsprechend kostspielige Neutralisations- und Abwasserreinigungsoperationen werden eingespart. Das Verfahren ist umweltfreundlicher. Alle diese vorteilhaften Ergebnisse sind im Hinblick auf den Stand der Technik überraschend. Man mußte im Hinblick auf die bekannten Verfahren verringerte Umsätze, Ausbeuten und die Bildung heterogener Gemische aus Ausgangs- und Endbase und ihren Salzen erwarten.

Der Ausgangsstoff II wird mit dem Ausgangsstoff III in stöchiometrischer Menge oder mit einem Überschuß einer Komponenten mit Bezug auf die andere, vorzugsweise mit einem Verhältnis von 0,1 bis 1,5, insbesondere von 0,9 bis 1,1 Mol Ausgangsstoff III je Mol Ausgangsstoff II, umgesetzt. Bevorzugte Ausgangsstoffe II und III und dementsprechend bevorzugte Endstoffe I sind solche, in deren Formeln die einzelnen Reste $R^1$, $R^2$ und $R^3$ gleich oder verschieden sein können und jeweils ein Wasserstoffatom, einen Alkylrest mit 1 bis 18, vorzugsweise 1 bis 6 Kohlenstoffatomen, einen Aralkylrest oder Alkylarylrest mit 7 bis 12 Kohlenstoffatomen, einen Phenylrest, einen Naphthylrest bedeuten, $R^2$ auch

ein Bromatom oder Chloratom, den Rest -CN oder den Rest $-O-R^4$ bezeichnet, $R^3$ auch für den Rest $-\overset{O}{\overset{\|}{C}}-R^4$ steht, $R^4$ einen Alkylrest mit 1 bis 18, vorzugsweise 1 bis 6 Kohlenstoffatomen, einen Aralkylrest oder Alkylarylrest mit 7 bis 12 Kohlenstoffatomen, einen Phenylrest oder einen Naphthylrest bedeutet. Die genannten Reste können noch durch unter den Reaktionsbedingungen inerte Gruppen, z.B. Alkylgruppen mit 1 bis 3 Kohlenstoffatomen, Cyangruppen, substituiert sein.

Anstelle von 1,3-Dicarbonylverbindungen II werden in der Regel Stoffe, die unter den Reaktionsbedingungen solche 1,3-Dicarbonylverbindungen II bilden, verwendet werden. Vorwiegend kommen Bisacetale, Monoacetale, Bisacylale und Monoacylale zur Anwendung. Zweckmäßig kommen Stoffe der Formel

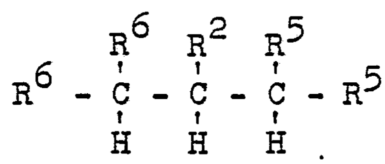 IV

oder 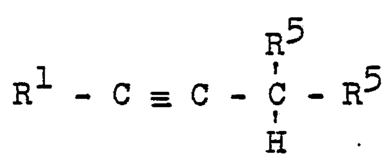 V,

oder 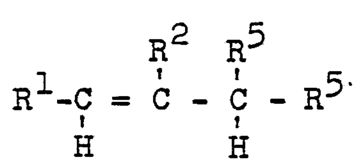 VI,

worin die einzelnen Reste $R^5$ und $R^6$ gleich oder verschieden sein können und jeweils den Rest $-OR^4$ oder den Rest $-O-\overset{O}{\overset{\|}{C}}-R^4$ bedeuten, die beiden Reste $R^5$ auch zusammen eine Oxogruppe bezeichnen, ein Rest $R^5$ auch für ein Halogenatom,

zweckmäßig ein Chloratom, stehen kann, $R^1$, $R^2$ und $R^4$ die vorgenannte allgemeine und bevorzugte Bedeutung besitzen, in Betracht.

Die Umsetzung läßt sich für den Fall der Verwendung von 1,1,3,3-Tetramethoxypropan und Hydrazin durch die folgenden Formeln wiedergeben:

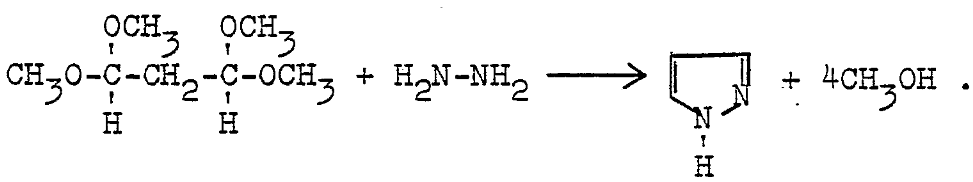

Geeignete Ausgangsstoffe II sind beispielsweise: Unsubstituierte oder durch die Methyl-, Äthyl-, Propyl-, Isopropyl-, Butyl-, Isobutyl-, sek.-Butyl-, tert.-Butyl-, Benzyl-, Phenyl-, Chlor-, Cyano-, Methoxy-, Äthoxy-, Propoxy-, Isopropoxy-, Butoxy-, sek.-Butoxy-, Isobutoxy-, tert.-Butoxy-, Benzoxy-, Phenoxy-gruppe in α-Stellung substituierte Tetramethoxy-, Tetraäthoxy-, Tetrapropoxy-, Tetraisopropoxy-, Tetrabutoxy-, Tetraisobutoxy-, Tetra-sek.-butoxy-, Tetra-tert.-butoxy-(1,1,3,3)-propane, 3,3-Dimethoxy-, 3,3-Diäthoxy-, 3,3-Dipropoxy-, 3,3-Diisopropoxy-, 3,3-Dibutoxy-, 3,3-Diisobutoxy-, 3,3-Di-tert.-butoxy-, 3,3-Di-sek.-butoxy-propionaldehyde, 1,1-Dimethoxy-, 1,1-Diäthoxy-, 1,1-Dipropoxy-, 1,1-Diisopropoxy-, 1,1-Dibutoxy-, 1,1-Diisobutoxy-, 1,1-Di-sek.-butoxy-, 1,1-Diisobutoxy-, 1,1-Di-tert.-butoxy-propanale; homologe, in α-Stellung unsubstituierte oder in vorgenannter Weise substituierte ß-Oxo-Butanale, ß-Oxo-Pentanale, ß-Oxo-Hexanale, ß-Oxo-Heptanale, ß-Oxo-Octanale; in α-Stellung zu beiden Carbonylgruppen unsubstituierte oder in vorgenannter Weise substituierte Pentan-2,4-dione, Hexan-2,4-dione, Heptan-2,4-dione, Octan-2,4-dione, Heptan-3,5-dione, Octan-3,5-dione; entsprechend in α-Stellung unsubstituierte oder in vorgenannter Weise sub-

stituierte, 1,1-acetalisierte, in α,ß-Stellung ungesättigte Butenale, Pentenale, Hexenale; 1,1-acetalisierte Propargylaldehyde und homologe in α,ß-Stellung ungesättigte Butinale, Pentinale, Hexinale.

Bevorzugt sind 1,1,3,3-Tetramethoxypropan, 1,1,3,3-Tetraäthoxypropan, 1,1,3,3-Tetraisobutoxypropan, 1,1,3,3-Tetraisopropoxypropan, 1,1,3,3-Tetrabutoxypropan, 1,1,3,3-Tetrapropoxypropan.

Als Hydrazine III kommen z.B. in Frage: Hydrazin, durch die Methyl-, Äthyl-, Propyl-, Isopropyl-, Butyl-, sek.-Butyl-, tert.-Butyl-, Isobutyl-, Benzyl-, Phenyl-, Acetyl-, Benzoyl-, Phenylacetyl-gruppe substituierte Hydrazine. Anstelle der Hydrazine III können auch unter den Reaktionsbedingungen diese Hydrazine III bildende Stoffe verwendet werden, z.B. die Hydrazinhydrate; Salze der Hydrazine wie die Sulfate oder Disulfonate, z.B. die Dibenzolsulfonate; Hydrazone, z.B. mit Formaldehyd, Acetaldehyd, zusammen mit Aminen wie Äthylamin. Im Falle von Salzen beträgt die Gesamtmenge an freier und an das Hydrazin gebundener Säure stets weniger als 2 Äquivalente Säure je Mol Hydrazin.

Die Umsetzung wird bei einer Temperatur von -10 bis +100, vorzugsweise von 0 bis 100°C, insbesondere von 30 bis 70°C, drucklos oder unter Druck, kontinuierlich oder diskontinuierlich durchgeführt. Gegebenenfalls setzt man unter den Reaktionsbedingungen inerte, organische Lösungsmittel zu. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Halogenkohlenwasserstoffe, insbesondere Chlorkohlenwasserstoffe, z.B. Tetrachloräthylen, 1,1,2,2- oder 1,1,1,2-Tetrachloräthan, Amylchlorid, Dichlorpropan, Methylenchlorid, Dichlorbutan, Tetrachlorkohlenstoff, 1,1,1- oder 1,1,2-Tri-

chloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, o-, p- und m-Dichlorbenzol, o-, m-, p-Chlortoluol, 1,2,4-Trichlorbenzol; Äther, z.B. Äthylpropyläther, Methyl-tert.-butyläther, n-Butyläthyläther, Di-n-butyläther, Diisobutyläther, Diisoamyläther, Diisopropyläther, Cyclohexylmethyläther, Diäthyläther, Äthylenglykoldimethyläther, Tetrahydrofuran, Dioxan; Alkanole und Cycloalkanole wie Äthanol, Methanol, n-Butanol, Isobutanol, tert.-Butanol, Glykol, n-Propanol, Isopropanol, Amylalkohol, Cyclohexanol, 2-Methyl-4-pentanol, Äthylenglykolmonoäthyläther, 2-Äthylhexanol, Methylglykol, insbesondere solche mit 1 bis 4 Kohlenstoffatomen; aliphatische oder cycloaliphatische Kohlenwasserstoffe, z.B. Heptan, Pinan, Nonan, Benzinfraktionen innerhalb eines Siedepunktintervalls von 70 bis 190°C, Cyclohexan, Methylcyclohexan, Dekalin, Petrläther, Hexan, Ligroin, 2,2,4-Trimethylpentan, 2,2,3-Trimethylpentan, 2,3,3-Trimethylpentan, Octan; und entsprechende Gemische. Zweckmäßig verwendet man das Lösungsmittel in einer Menge von 400 bis 10 000 Gewichtsprozent, vorzugsweise von 100 bis 500 Gewichtsprozent, bezogen auf Ausgangsstoff II.

Stets wird Wasser, zweckmäßig in einer Menge von 1 bis 70, vorteilhaft von 1 bis 10 Mol Wasser, bezogen auf Hydrazin III, verwendet. Es wird vorteilhaft zusammen mit einer anderen Reaktionskomponente, z.B. mit der Säure und/oder dem Ausgangsstoff III, dem Ausgangsgemisch zugeführt.

Die Reaktion wird in Gegenwart von weniger als 2 Äquivalenten Säure, vorteilhaft mit einer Menge von 0,05 bis 1,9, insbesondere von 0,25 bis 1,25 Äquivalenten Säure, bezogen auf 1 Mol Ausgangsstoff III, durchgeführt. Es können anorganische oder organische Säuren verwendet werden. Anstelle einbasischer Säuren können auch äquivalente Mengen mehrba-

sischer Säuren zur Anwendung gelangen. Beispielsweise sind folgende Säuren geeignet: anorganische Säuren wie Chlorwasserstoff, Bromwasserstoff, Schwefelsäure, Phosphorsäure; Sulfonsäuren wie Benzol- und p-Toluolsulfonsäure; Bor enthaltende Säuren wie Borsäure, Borfluorwasserstoffsäure; aliphatische Carbonsäuren wie Chloressigsäure, Dichloressigsäure, Trichloressigsäure, Oxalsäure, Ameisensäure, Essigsäure, Propionsäure, Buttersäure, Isobuttersäure, Glykolsäure, α- bzw. ß-Chlorpropionsäure; oder entsprechende Gemische. Die Säuren können in konzentrierter Form, im Gemisch miteinander und/oder mit einem Lösungsmittel, insbesondere Wasser, angewendet werden. Bevorzugt ist Salzsäure. Der pH der Umsetzung beträgt zwischen 0 und 6,9, vorzugsweise von 0,5 bis 4, insbesondere von 0,9 bis 2,5.

Die Reaktion kann wie folgt durchgeführt werden: Ein Gemisch von Hydrazin III, Säure und Wasser wird auf das Reaktions-pH gebracht und dann gleichzeitig weitere Ausgangsstoffe III und Ausgangsstoff II, gegebenenfalls zusammen mit Wasser und/oder organischem Lösungsmittel so zugegeben, daß das Reaktions-pH stets eingehalten wird. Vorteilhaft sind eine Zugabetemperatur von -10 bis +60°C und Zugabezeiten von 5 bis 30 Minuten. Die gesamte Reaktionszeit beträgt vorteilhaft 0,1 bis 12 Stunden. Es zeigt sich als wesentlicher Vorteil des erfindungsgemäßen Verfahrens, daß lediglich minimale Mengen an Säureäquivalenten, bezogen auf das Hydrazinderivat, insgesamt zugesetzt werden müssen. Aus dem Reaktionsgemisch kann der Endstoff in üblicher Weise, z.B. durch Neutralisation und Extraktion des Gemischs und Destillation des Extrakts, abgetrennt werden. Die Neutralisation kann bei diskontinuierlicher Fahrweise z.B. durch eine anorganische Base erfolgen, wobei erfindungsgemäß nur noch soviel Salz als Nebenprodukt anfällt, als der vermindert eingesetzten Säure entspricht. Insbesondere bei kontinuierlicher Fahrweise aber erfolgt die Freisetzung der

Pyrazole I erfindungsgemäß durch das Zufügen des entsprechenden Hydrazins III zum Reaktionsgemisch, wobei vorteilhaft vor der Extraktion pH-Werte von 6 bis 10 eingestellt werden. Wie folgendes Formelschema am Beispiel des Pyrazols zeigt, wird so wieder einsatzfähiges Hydrazinsalz in Lösung zurückerhalten:

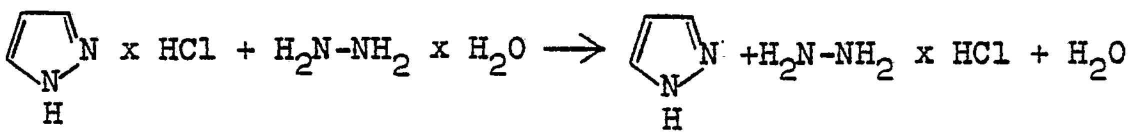


Verluste an Säureäquivalenten treten vorteilhaft nur noch insoweit auf, als sie bei Destillations- und Extraktionsvorgängen durch Übertritt aus der wäßrigen Phase entstehen. Die Freisetzung des Endstoffs I durch zusätzliches Hydrazin erfolgt zweckmäßig bei 0 bis 40°C. Auf vorgenannte Weise kann die entstehende Hydrazinsalzlösung in beliebig wiederholten Umsetzungen wiederverwendet werden, was ein wesentlicher und überraschender Vorteil des Verfahrens darstellt.

Die nach dem Verfahren der Erfindung herstellbaren Pyrazole I sind wertvolle Ausgangsstoffe für die Herstellung von Farbstoffen, Pflanzenschutzmitteln und Pharmazeutika. Bezüglich der Verwendung wird auf vorgenannte Veröffentlichungen verwiesen.

Die in den folgenden Beispielen angegebenen Teile bedeuten Gewichtsteile. Die Gewichtsteile verhalten sich zu den Volumenteilen wie Kilogramm zu Liter.

<u>Beispiel 1</u>

In einer Rührapparatur werden 320 Teile Wasser und 20 Teile (0,4 Mol) Hydrazinhydrat vorgelegt und bei 40°C durch Zugabe von 59 Teilen wäßrige, 36-gewichtsprozentige Salz-

säure (0,5 Mol) auf pH 1 gestellt. Im Verlauf von 2 1/2 Stunden werden aus drei Vorratsgefäßen gleichzeitig (392 Volumenteile) 380 Teile (7,6 Mol) Hydrazinhydrat, 354 Teile wäßrige, 36-gewichtsprozentige Salzsäure (3,54 Mol) und 1 312 Teile (8 Mol) 1,1,3,3-Tetramethoxypropan portionsweise zugegeben, so daß ein pH von 2 bis 3 aufrechterhalten bleibt. (43 bis 55°C ohne Kühlung). Dabei wird die Zulaufgeschwindigkeit des 1,1,3,3-Tetramethoxypropan bei 540 Volumenteilen pro Stunde konstant gehalten und der Hydrazinzulauf auf 153 Teile pro Stunde eingestellt. Der pH wird über die Salzsäurezugabe reguliert. Nach einer Nachrührzeit von 7 Stunden wird das Gemisch durch Zugabe von 320 Teilen (4 Mol) NaOH auf pH 8 gestellt und 1 553 Teile Methanol/Wassergemisch anschließend bei 20 mbar/50°C in 2 1/2 Stunden abdestilliert. Der Rückstand wird mit 2 000 Volumenteilen Methylenchlorid aufgerührt und abgesaugt. Man erhält im Filtergut 250 Teile NaCl und aus dem Filtrat nach der Destillation 445 Teile (82 % der Theorie) Pyrazol vom Kp 60 bis 70°C (0,3 mbar).

Beispiel 2

a) In einer Rührapparatur mit pH-Elektrode werden 12,9 Teile (0,258 Mol) Hydrazinhydrat vorgelegt und mit 50,6 Teilen wäßrige Salzsäure (0,506 Mol) unter Kühlung neutralisiert. Es fällt zunächst Hydrazinhydrochlorid aus. Jetzt werden bei 45 bis 50°C 1,1,3,3-Tetramethoxypropan und weiteres Hydrazinhydrat gleichzeitig so zugegeben, daß ein pH von 1 bis 2 erhalten bleibt, nämlich während 20 Minuten 82 Teile (0,5 Mol) 1,1,3,3-Tetramethoxypropan

und 15,5 Teile (0,31 Mol) Hydrazinhydrat. Nach beendeter Zugabe beträgt das pH 1. Nun wird das Gemisch 2 Stunden bei 22°C nachgerührt.

b) 120 Volumenteile (= 3/4) dieser Lösung werden jetzt mit 20,6 Volumenteilen (0,426 Mol) Hydrazinhydrat auf pH 6,8 bis 7,2 gestellt und anschließend mit 2 x 200 Volumenteilen $CH_2Cl_2$ extrahiert. (Hydrazinhydratphase/Extrakt). Durch Destillation des Extraktes erhält man 23,2 Teile Pyrazol (85 % der Theorie) vom Kp 60 bis 70°C/0,1 mbar).

c) Jetzt wird das restliche Viertel, nämlich 40 Volumenteile der in Beispiel 2a) erhaltenen Reaktionslösung (pH: 1), vorgelegt und während 20 Minuten analog Beispiel 2a mit 61,5 Teilen (0,375 Mol) 1,1,3,3-Tetramethoxypropan und der in b) extrahierten, wäßrigen Hydrazinhydratphase (pH 6,9 bis 7,2) versetzt. Es stellt sich wieder ein pH von 1 bis 2 ein. Nach weiteren 2 Stunden wird das Gemisch in gleicher Weise wie in Beispiel 2b) aufgearbeitet. Man erhält nach Destillation 21,45 Teile (0,36 Mol) Pyrazol (entsprechend 84 % Ausbeute) vom Kp 60 bis 70°C (0,1 mbar).

d) Diese Arbeitsweise c) wird bei weiteren 10 Umsetzungen wiederholt durchgeführt. Man erhält noch im 10. Durchgang ein pH des Reaktionsgemisches von 1,6 bis 1,7 und dieselben Ergebnisse wie in Beispiel 2c).

## Patentansprüche

1. Verfahren zur Herstellung von Pyrazolen der Formel

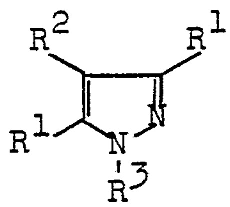

I,

worin die einzelnen Reste $R^1$, $R^2$ und $R^3$ gleich oder verschieden sein können und jeweils ein Wasserstoffatom, einen aliphatischen, araliphatischen oder aromatischen Rest bedeuten, $R^2$ auch ein Halogenatom, den Rest -CN oder den Rest -O-$R^4$ bezeichnet, $R^3$ auch für den Rest 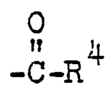 steht, $R^4$ einen aliphatischen, araliphatischen oder aromatischen Rest bedeutet, durch Umsetzung von 1,3-Dicarbonylverbindungen mit Hydrazinen in Gegenwart von Wasser und Säure, dadurch gekennzeichnet, daß man 1,3-Dicarbonylverbindungen der Formel

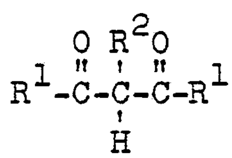

II,

worin $R^1$ und $R^2$ die vorgenannte Bedeutung besitzen, mit Hydrazinen der Formel

$R^3$-HN-$NH_2$ III,

worin $R^3$ die vorgenannte Bedeutung besitzt, in Gegenwart von weniger als 2 Äquivalenten Säure je Mol Hydrazin III bei einer Temperatur von -10 bis +100°C umsetzt, wobei der pH-Wert des Reaktionsgemischs während der Reaktion zwischen 0 und 6,9 beträgt.

2. Verfahren nach Anspruch 1, dadurch gekennzeichnet, daß man in dem Reaktionsgemisch den Anteil des Pyrazols I in Gestalt des Pyrazolsalzes mit zusätzlichem Hydrazin III zu freiem Pyrazol I umsetzt.

0020964

Europäisches Patentamt

# EUROPÄISCHER RECHERCHENBERICHT

Nummer der Anmeldung

EP 80 10 2515

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | FR - A - 2 230 637 (PRODUITS CHIM. UGINE KUHLMANN)<br>* Beispiel 1, Seiten 3,4 *<br>-- | 1,2 |
| | US - A - 3 200 128 (H.A. WAGNER)<br>* Spalten 1-4 *<br>-- | 1,2 |
| | GB - A - 641 015 (GENERAL AIRLINE)<br>* Seiten 2,3 *<br>---- | 1,2 |

KLASSIFIKATION DER ANMELDUNG (Int. Cl.³)

C 07 D 231/12
231/14
231/16
231/18

RECHERCHIERTE SACHGEBIETE (Int. Cl.³)

C 07 D 231/12
231/14
231/16
231/18

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 22-09-1980 | CREMERS |

EPA form 1503.1 06.78